(19) Europäisches Patentamt / European Patent Office / Office européen des brevets

(11) **EP 4 463 058 B1**

(12) **EUROPEAN PATENT SPECIFICATION**

(45) Date of publication and mention
of the grant of the patent:
**18.03.2026 Bulletin 2026/12**

(21) Application number: **22701910.6**

(22) Date of filing: **14.01.2022**

(51) International Patent Classification (IPC):
***A61B 5/00*** *(2006.01)*

(52) Cooperative Patent Classification (CPC):
**A61B 5/0095; A61B 2560/0443; A61B 2562/185**

(86) International application number:
**PCT/EP2022/050721**

(87) International publication number:
**WO 2023/134859 (20.07.2023 Gazette 2023/29)**

(54) **PHOTO-ACOUSTIC IMAGING DEVICE AND TRANSDUCER MOUNTING METHOD**

PHOTOAKUSTISCHE BILDGEBUNGSVORRICHTUNG UND WANDLERMONTAGEVERFAHREN

DISPOSITIF D'IMAGERIE PHOTO-ACOUSTIQUE ET PROCÉDÉ DE MONTAGE DE TRANSDUCTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.11.2024 Bulletin 2024/47**

(73) Proprietor: **PA Imaging Holding B.V.**
**7522 NM Enschede (NL)**

(72) Inventors:
• **VAN MEERDERVOORT, Rutger, Pieter, Pompe**
**7522 NM Enschede (NL)**
• **ALINK, Laurens**
**7522 NM Enschede (NL)**
• **OP'T ROOT, Timotheus, Johannes, Petrus, Maria**
**7522 NM Enschede (NL)**

(74) Representative: **V.O.**
**P.O. Box 87930**
**2508 DH Den Haag (NL)**

(56) References cited:
US-A1- 2010 053 618    US-A1- 2015 208 924
US-A1- 2017 030 866    US-A1- 2017 059 530
US-A1- 2018 106 767    US-A1- 2018 292 358
US-A1- 2019 046 042    US-A1- 2019 150 749
US-A1- 2019 275 562

**Description**

Field of the invention

This invention relates to a photo-acoustic imaging device and a method of mounting an acoustic-electronic transducer in a photo-acoustic imaging device, e.g. when the photo-acoustic imaging device is assembled.

Background

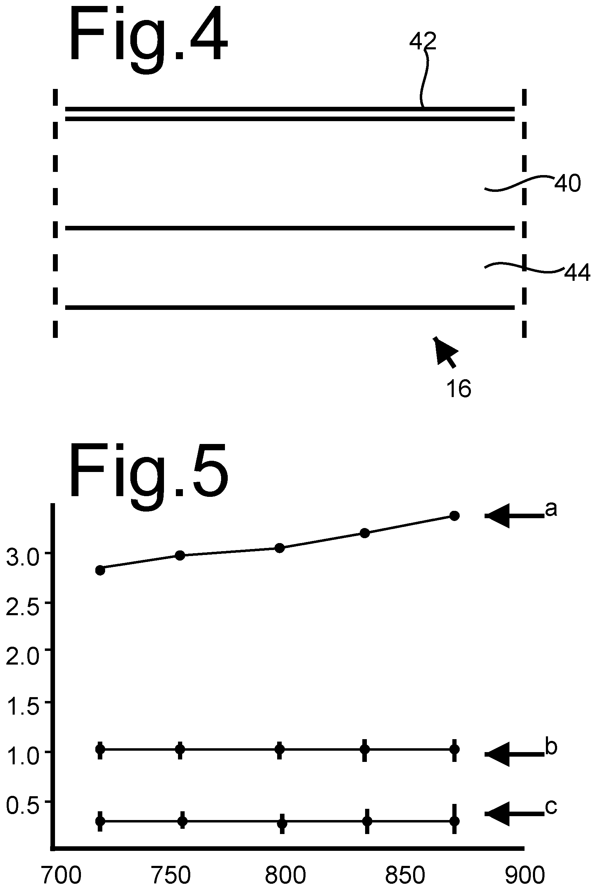

Photo-acoustic imaging involves sending light pulses into tissue and formation of an image (usually a 3 D image) of sound generation due to absorption of the light in the tissue (as used herein "sound" covers "ultrasound"). The tissue is presented in a basin that also contains an acoustic contact liquid such as water.
The pulses are preferably very short (nanosecond) and powerful (near-) infrared pulses, provided by a light source that has an output or distributed light outputs on the wall of the basin.
In the tissue, the light pulses give rise to light absorption, leading to an increase in temperature which causes thermal expansion which in turn leads to pressure transients that give rise to low intensity sound waves (especially from blood). An array of ultrasensitive broadband acoustic to electronic transducers in openings through the wall of the basin detects these waves.
The detection results can be used to reconstruct an image e.g. a complete 3D-blood vessel system, with high spatial resolution.
The detected sound wave intensity is very small, especially from photo-acoustic conversion in deeper parts of the tissue that further off from the surface positions (at the skin) where the light is supplied.
This makes it necessary to be able to distinguish tiny photo acoustic responses from deep(er) lying tissue like blood vessels from noise and artifacts in 3D image reconstructions of these blood vessels. This places high demands on suppression of artifacts that disturb small ultrasound signals. It has been found that disturbing artifacts can be due to light that reaches the sound receiving surface of the acoustic to electronic transducers and causes photo-acoustic conversion at that surface.
The direct (ultra-) sound signal from such photo-acoustic conversion can easily be eliminated by time windowing, since this direct signal precedes the arrival of sound waves from the tissue that have to travel from positions of photo-acoustic conversion within the tissue.
But sound generated at the sound receiving surface of the acoustic to electronic transducer can also travel into the tissue and get reflected from there to the acoustic to electronic transducers. It has been found that artifacts due to such reflection are much harder to eliminate by signal processing. Instead, the inventors have tried to reduce photo-acoustic auto-response of the surface of the acoustic to electronic transducer. A possible way to reduce the photo-acoustic response from this surface is to coat the surface with a light reflecting layer that reflects the (near-) infrared light, minimizing light that reaches into the coating and the surface underneath the coating where photo-acoustic can occur, without significantly diminishing sound transmission from the tissue to the surface of the acoustic to electronic transducer.
A gold coating may be used for example. Gold has an additional beneficial property that it is inert, especially to oxygen, thereby preserving the reflecting surface. It is known that gold is a very effective reflector for (near-)infrared light. See ref. [1] Paquin, R.E. "Properties of Metals" in: Bass. M (eic.) Handbook of Optics, Volume II Devices, Measurements, and Properties, Chapter 35, pages 35.1 - 35.74, McGraw-Hill Professional, New York, second edition (1995); ISBN 0-07-047974-7, TABLE 3 Reflectance of Selected Metals at Normal Incidence (page 35.33-34). See also ref. [2] Lynch, D.W., "Mirror and Reflector Materials , " in: Weber, M.J. (ed . ), CRC Handbook of Laser Science and Technology, IV, Optical Materials, Part 2 : Properties, CRC Press, Boca Raton, Florida (2003); ISBN 0-8493-3512-4, Section 1.3, Fig. 4.2.8.
In principle, a very thin (sub-micrometer (e.g. a few hundred nanometer)) reflective gold coating may suffice to prevent significant light transmission to the underlying surface of the acoustic to electronic transducer. This is advantageous, because it can be used to ensure that the gold layer is thin enough to avoid loss of transmitted ultrasound wave intensity e.g. due to reflection.
But it has been found that, although the gold layer has a minimal thickness that is sufficiently reflective, artifacts like those due to the underlying surface of the acoustic to electronic transducer still arise. It has been found that, without a significantly thicker gold coating than needed for reflection, a gold coating on the surface of the acoustic to electronic transducer only poorly reduces artifacts like those of photo-acoustic conversion at the surface of the acoustic to electronic transducer.
A sufficient thickness of this coating would be required to obtain a coating that overcomes the roughness of the sound receiving surface of the transducer and cover the entire area with a sufficiently thick layer of gold. When vapor deposition or sputtering is used to deposit such a gold layer, the layer will suffer from shadow effects, when the treated surface is not entirely smooth, resulting in the phenomenon that crevices and indentations in the surface will not have (sufficient) thickness of gold everywhere, which would result in a situation in which light can still be absorbed on the surface in areas that are not (sufficiently) covered. As a result, undesired photo-acoustic signals can still be generated which in turn will adversely affect the Signal-to-Noise Ratio (SNR) of the system.

To overcome this, either the sound receiving surface could be pretreated to obtain a sufficiently smooth surface and thus a homogeneous reflective layer, or a relatively thick layer of reflective material needs to be applied, which layer on the other hand should be thin enough to be fully transparent for ultrasound.

US2019/275562 discloses an ultrasound transducer with a ground electrode on a membrane over the receiving surface of the transducer, with the ground electrode connected to metallic housing of the transducer. This is used to prevent electromagnetic interference, e.g. from RF fields that are used to create acoustic signals from a sample by heating the sample. Metal rubber sheets are known that can function as a conductive membrane. In principle, a membrane with a conductive layer could also be used for ultrasound transducers in a photo-acoustic imaging system, in which case the ground electrode on the membrane could reflect some of the light that could reach the surface of the acoustic to electronic transducer. However, such a membrane construction does not guarantee a combination of high transmission of the weak photo-acoustic signals from the sample and high reflection of the light from the light pulses.

It is noted that patent publication US 2017/0030866 A1 discloses a photoacoustic apparatus including a holding unit that holds a subject, a light irradiation unit that irradiates a subject with light via the holding unit, and a probe that receives an acoustic wave propagated from the subject, wherein the holding unit includes a light diffusion surface that diffuses the light and of which a degree of diffusion of the light is lowered when an acoustic matching material is applied thereto.

It is further noted that patent publication US 2018/292358 A1 discloses a probe array including a plurality of probes each having a reception surface which comes in contact with the acoustic matching liquid stored in a vessel, and a support portion that supports the plurality of probes and has a proximal surface which comes in contact with the acoustic matching liquid and is adjacent to the reception surface. The reception surface is equal or smaller in a contact angle with respect to the acoustic matching liquid to or than the proximal surface.

It is also noted that patent publication US 2017/059530 A1 discloses an acoustic wave probe including a support member having a plurality of through holes and a concave portion which is concave facing an object disposed in a measurement position at the time of measurement, and an acoustic wave transducer unit having at least a transducer. The acoustic wave transducer unit is mounted in the through hole facing approximately a center of curvature of the concave portion, and a thickness of the acoustic wave transducer unit is thinner on a side of the center of curvature.

Summary

It is an object to reduce artifacts due to photo-acoustic conversion at transducers used in a photo-acoustic imaging device (e.g. for mammography applications).

According to an aspect, reduction of the artifacts due to the photo-acoustic response of the transducers is achieved by the photo-acoustic imaging device comprising

- a basin for receiving tissue to be imaged;
- a light source configured to supply light to an interior of the basin;
- a plurality of acoustic-electronic transducers, each transducer of said plurality having a sound receiving surface that forms part of the surface of the basin, at a respective different position in the surface of the basin, each with a foil adhesively connected to the receiving surface and a reflective coating provided on the basin side of the foil. The use of a foil, such as a metal foil, or more specifically a copper foil with a reflective coating such as a gold coating, and an adhesive makes it easy to install and to repair the photo-acoustic imaging system in a way that achieves the high demands on suppression of artifacts in the context of a photo-acoustic imaging device. Such a foil can even be installed after the transducers are mounted in the basin, during initial assembly or repair.

Preferably, a robust highly reflective material such as gold is used for the reflective coating.

It has been found that, when a reflective coating that is reflective for the light from the light source (e.g. in the near infrared), such as a gold coating, on the basin-side surface of a smooth flexible metal foil such as a copper foil is used, a reflective coating suffices to reduce artifacts in a way that makes sensitive photo-acoustic imaging better possible, compared to when a reflective surface is used directly on the surface of the acoustic to electronic transducer. It is assumed that this is because the metal foil makes it possible to improve the smoothness of the reflective coating, compared to when the coating would be applied directly on the transducer surface, which in turn reduces photo-acoustic conversion due light absorption by the reflective coating as a result of multiple reflections by the reflective coating. Preferably, a patch is used wherein the coating surface is so smooth that its RMS slope value is less than 0.42 and more preferably at most 0.1. Preferably the foil surface underlying the coating also has an RMS slope value of less than 0.42 and more preferably at most 0.1 The smoothness of metal foils that are manufactured using cold rolling, for example, can be sufficient to improve the smoothness of the reflective coating. Alternatively, a polymer foil with comparable smoothness may be used.

Preferably a gold coating on a copper layer is used. Gold is a good infrared and near infrared light reflector. Furthermore, gold and copper layers hardly reduce ultra-sound transmission to the ultrasound transducer, and these layers can be made thin to minimize the reduction of sound transmission. Moreover, gold is resistant to degradation of the light reflection and copper makes the smoothness more robust.

In an embodiment, the smooth foil may be attached to the sound receiving surface of the transducer by means of an adhesive layer, which may be provided on the transducer-side surface of the foil before the foil is applied to the sound receiving surface of the transducer. The adhesive is chosen for ease of application and it is chosen such that it is possible to easily remove the coated foil from the transducer, when either the foil or the transducer needs replacement. The smooth foil may be provided in the form of a self-adhesive tape.

The acoustic-electronic transducers each have an electrically conductive housing around an axis normal to the receiving surface. In an embodiment, the electrically conductive housing is electrically connected to the metal foil. Thus, the metal foil can perform two functions: reduction of photo-acoustic signal generation and shielding of electromagnetic fields at the transducers.

In addition to the functional benefits, the cost of the application of the reflective material is also significantly reduced, because of this it is less costly to apply a reflective material on a foil than on a transducer. It is much easier and effective to position a foil in a vapor deposition chamber compared to a complete transducer element.

In an embodiment wherein the transducers extend through openings of a basic structure of the basin, the copper foil is provided in patches on each of said plurality of acoustic-electronic transducers, extending from the receiving surfaces of the acoustic-electronic transducer to the surface of the basic structure around the opening through which the acoustic-electronic transducer extends. A gap between the transducer and the basic structure may contain an O-ring to prevent water from leaking from the basin or other material. However, it has been found that the gap or material in the gap may contribute to photo-acoustic noise sound generation. The use of a gold coated copper patch that extends from the sound receiving surface of the transducer to the surface of the basic structure around the transducer over the gap can prevent, or at least reduce, photo generation of acoustic noise sound generation in the gap.

The edge of the patch can also give rise to some photo-acoustic noise sound generation, but it has been found that the intensity of such sound is smaller than that from the gap.

Preferably, different ones of the plurality of acoustic-electronic transducers each have their own foils with a reflective coating in the form of mutually separate patches. When the surface of a transducer is shallowly damaged, for instance after disinfecting the wall of the measurement basin in which the transducers are mounted, or gets worn from such cleaning, the patch can be easily removed and replaced by another patch without having to dismount and replace a complete transducer element, which is much more expensive and takes much more labor time to replace than replacing a patch.

Brief description of the drawing

These and other objects and advantageous aspects will become apparent from a description of exemplary embodiments with reference to the following figures.

Figure 1 shows a basin of a photo acoustic imaging device
Figure 2 s chematically illustrates part of the basin of a photo acoustic imaging device
Figure 3 schematically illustrates a top view of an acoustic-to-electronic transducer
Figure 4 shows a cross-section of a patch
Figure 5 shows exemplary normalized RMS ultrasound signal amplitudes
Figure 6 shows an embodiment wherein an electrically conductive layer

Detailed description of exemplary embodiments

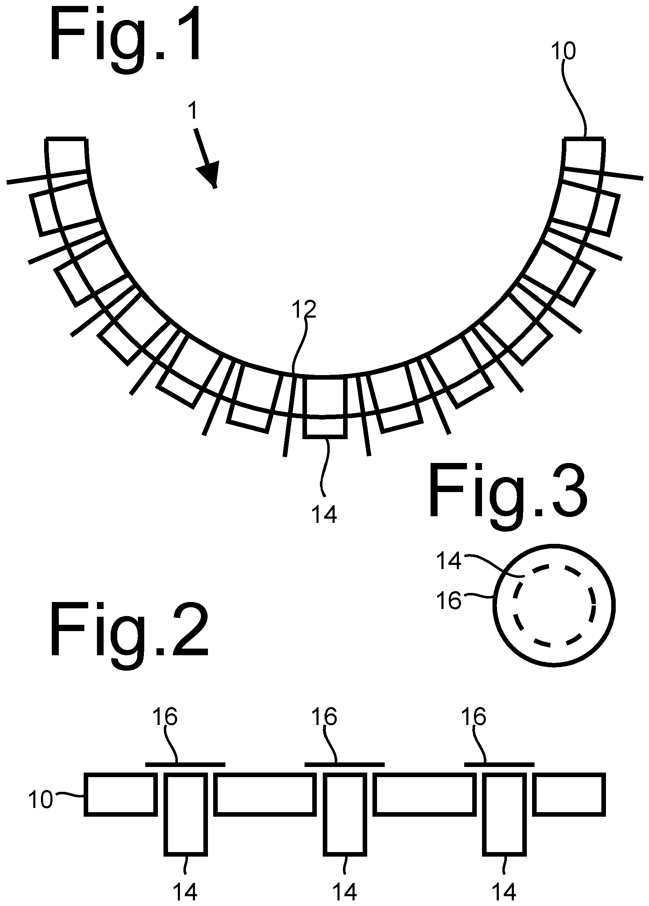

Figure 1 shows a basin of a photo acoustic imaging device in the form of a hemispherical bowl 1 that has an interior for receiving a breast (not shown) in vivo. The surface of bowl 1 is defined by a basic structure 10 with one or more outputs 12 of a light source for supplying light into bowl 1 and openings for acoustic-to-electronic transducers 14. Although a hemispherical bowl 1 for receiving a breast is shown by way of example, it should be appreciated that other kinds of tissue may be imaged and that a differently shaped basin may be used.

Figure 2 schematically illustrates part of the basin of a photo acoustic imaging device, including basic structure 10 (shown flattened to simplify the illustration) with acoustic-to-electronic transducers that extend through the openings.

Figure 3 schematically illustrates a top view of an acoustic-to-electronic transducer 14. Patches 16 of gold coated copper foil are provided at least above acoustic-to-electronic transducers 14. Patch 16 covers the sound receiving surface of acoustic-to-electronic transducer 14. Preferably, patch 16 extends onto basic structure 10 around the opening that contains acoustic-to-electronic transducer 14, as shown.

Figure 4 shows a cross-section of a patch 16 (not to scale) with a copper foil 40, a gold coating 42 on a basin side surface of copper foil 40 and an adhesive layer 44 on a transducer side surface of patch 16. In an exemplary embodiment an 18 micrometer thick copper foil was used, with a 0.5 micrometer thick gold coating. Preferably a nickel diffusion barrier layer may be used between copper and gold layers, or a NiCr, Cr layer instead of the nickel diffusion barrier. Other examples include cobalt, palladium, copper-tin alloys and alloys of such materials. The bottom of the copper foil attached to the surface of acoustic-to-electronic transducers 14 by a 32 micrometer thick acrylic adhesive. However, a copper foil thickness of between 1 and 100 micrometer may be used and a gold coating thickness between 0.1 and 2 micrometer and more preferably between 0.2 and 2 micrometer. The coating layer may be applied e.g. by electro-plating or other deposition methods such as sputtering or vapor deposition. Instead of a metal foil, a polymer foil with similar smoothness may be used. In an example, a 25 micrometer thick polyimide foil was used, with a 0.4 micrometer thick gold coating. Optionally, a thin Ti layer may be used as adhesion promotor between the Polyimide and gold on top of the polyimide foil, with the bottom of the foil attached to the surface of acoustic-to-electronic transducers 14 by a 45 micrometer thick silicone adhesive.

The thickness of the adhesive layer is not critical and it may vary slightly between different positions, but the adhesive layer can be optimized for ultrasound matching, for example by combining the adhesive layer with a layer on the transducer surface. See "Matching ultrasonic transducer using two matching layers where one of them is glue", by Callens et al. in NDT&E International 37 (2004) 591-597).

The thickness of the combination of the coating, the metal layer and the adhesive layer is preferably kept so low that ultrasound absorption and reflection by the combination does not significantly reduce the ultrasound signal strength at the transducer. For example, the thickness of the combination may be less than 100 micrometer, for example a layer of about 50 micrometer of about 1 micrometer gold, two micrometer nickel, 18 micrometer copper and 32 micrometer acrylic adhesive.

Although an embodiment is described wherein each acoustic-to-electronic transducer 14 has its own, separate patch, it should be appreciated that instead patches may be used that cover more than one acoustic-to-electronic transducer 14 in the basin wall, or even all of these acoustic-to-electronic transducers 14.

But for gaps, the surface of basic structure 10 of the bowl and the top surface of acoustic-to-electronic transducer 14 form a quasi-continuous surface, substantially without steps between basic structure 10 and the top surface of acoustic-to-electronic transducer 14 (e.g. with steps of less than 0.1 mm high). At most a small gap (e.g. less than 0.1 mm wide) is present between acoustic-to-electronic transducer 14 and basic structure 10. An O-ring or other means to prevent water from leaking from the basin may be provided in the gap. The top surface of acoustic-to-electronic transducer 14 acts as sound receiving surface.

The acoustic-to-electronic transducers may be installed in the photo-acoustic imaging device by- mounting each acoustic-electronic transducer at a position where the acoustic-electronic transducer extends through the opening, so that a sound receiving surface of the acoustic-electronic transducer forms part of the surface of the basin. Subsequently the foil may be attached to the sound receiving surface. This may be done for all of the acoustic-electronic transducers. The (patches of) foil may be provided with an adhesive layer on the surface opposite the surface with the gold coating, to attach the foil to the acoustic-electronic transducer.

Transducers with an acoustic matching layers on their sound receiving surface may be used. Efficient gold deposition on some acoustic matching layers is not possible, but by attaching a foil with a gold coating this can be overcome. When the foil is attached to the sound receiving surface with an acoustic matching layer this avoids that the entire transducer, or at least its active element, needs to be treated as a whole to apply the reflective material directly on the entire sound receiving surface of the transducer.

If needed, the foil with the gold coating over one or more the acoustic-electronic transducers may be removed later, and replaced by a new foil. When the foil is attached to the sound receiving surface (initially or as a replacement) foil is preferably attached so that it extends from the receiving surface of one or more acoustic-electronic transducer to the surface of the basic structure around the opening through which the one or more acoustic-electronic transducers 14 extend.

In operation, the light source transmits a light pulse through its outputs 12 into bowl 1 and acoustic-to-electronic transducers 14 receive sound generated in the interior of bowl 1. The generated sound comprises sound generated due to absorption of the light pulse. Acoustic-to-electronic transducers 14 receive the sound via the sound receiving surface of acoustic-to-electronic transducers 14 and convert the received sound into electronic signals. Acoustic-to-electronic transducers 14 may use piezo-electric material for this purpose. The photo acoustic imaging device comprises a data processing system (not shown, e.g. a computer) coupled to electronic outputs of acoustic-to-electronic transducers 14, and configured (e.g. programmed) to compute the amplitude of generated sound as a function of position in the interior of bowl 1. Methods for performing such computations are known per se. The resulting amplitude of generated sound as a function of position may be represented in the form of a 3D or 2D image.

Figure 5 shows results of experiments with surfaces of transducers that have different exemplary surface configurations. Ultrasound signal levels due to photoacoustic conversion at the surfaces of the transducers are shown. The transducers were mounted in a holder that mimicked a basin. Light pulses of varying near infrared wavelength were supplied to the transducers in the holder. A 3D photoacoustic imager was used to measure the ultrasound signals that were induced by irradiating the transducers.

The signals levels were determined in 3D photoacoustic reconstructed images, by computing the root mean square (RMS) of the reconstructed initial pressure levels over thin (2.4 mm) cylindrical volumes centered at the centers of the surfaces of the transducers and extending radially (7.2 mm) over the radius of a patch (e.g. 6 mm),which is larger than the 5 mm radius of the transducer surface, to capture all of the generated signals from the patch. The signal levels have been normalized for each wavelength to the average signal levels of the directly deposited surfaces (b).

The different curves are obtained with an acoustic-to-electronic transducer 14 covered by a patch of copper foil with a gold coating (c), an acoustic-to-electronic transducer 14 with a gold coating directly on the surface of acoustic-to-electronic transducer 14 (b) and an acoustic-to-electronic transducer 14 without gold coating (a).

As can be seen patches 16 of gold coated copper foil (c) result in a significant photoacoustic amplitude reduction. The transducer without reflective layer (a, i.e. wherein the sound receiving surface is directly exposed to the irradiated light), generates over 2.6 times larger signal levels compared to transducers with surfaces onto which a 1 micrometer gold layer was directly deposited via sputter deposition (b). Transducers with a gold coated copper patch applied generate signal levels that are further reduced by a factor 2.8 on average.

Although a theoretical explanation is not necessary to carry out such reductions, it may tentatively be assumed that the reduction of photoacoustic amplitude reduction is due to a reduction of multiple reflection from the gold coating (and its associated absorption) as a result of improved smoothness of the gold coating when the gold coating is provided on the copper foil rather than directly on an acoustic-to-electronic transducer 14. In turn, the improved smoothness of the gold coating is due to the fact that the copper foil makes it easy to provide a smoother substrate for the gold coating than the acoustic-to-electronic transducer 14.

Bergstrom, in Journal of Applied Physics 103, 103515 (2008): "The absorption of light by rough metal surfaces_ a three-dimensional ray-tracing analysis" shows simulation results of the relation between reflection and surface smoothness (expressed by means of surface roughness).To express this relation, the surface roughness can be characterized by means of the root mean square (RMS) slope of a surface (square root of the spatial average of the sum of the squares of $dh/dx$ and $dh/dy$, with h the height of the surface and $dh/dx$ and $dh/dy$ the derivatives of the height along different directions on the surface). Assuming a a Gaussian height distribution the RMS slope equals

$$\text{Sqrt}(2) * \text{sigma} / L$$

Wherein sigma is the root mean square of the height h and L is the correlation length of the height (cf. Bergstrom). The RMS slope can be determined by a profilometer, e.g. an optical profilometer observation through a microscope. For example, the RMS slope can be determined via the $R\Delta q$ parameters measured using a profilometer, in accordance with definitions and methods in [ISO 3287:1997]. It should be noted that the $R\Delta q$ values are a factor of square root two larger than the RMS slope definition used in Bergstrom.

It has been found from simulations with perpendicularly, or near perpendicularly incident light (deviating less than thirty degrees from the perpendicular) multiple reflections that are relevant for causing photo-acoustic signal generation at the surface can be substantially prevented, or at least significantly reduced if

$$\text{RMS slope} < 0.42 \ (\text{approximately } 0.3 * \text{sqrt}(2) )$$

Above this value light absorption due to multiple reflections increase significantly. An RMS slope of 0.42 can be extrapolated to result in a normalized RMS photoacoustic signal of about 0.35 in configurations wherein a gold coating is used. As can be seen from figure 5, a gold layer deposited on the transducer without copper foil results in a much higher photoacoustic signal due to light absorption.

More preferably this RMS slope is at most 0.1. In the case of a thin coating layer on an underlying foil, this maximum value formally applies to the surface of the coating layer. But if the underlying foil at most has this maximum RMS slope value, this usually ensures that the RMS slope of the coating is also smaller than this maximum value. The RMS slope of a thin coating layer is substantially the same or slightly lower than that of the underlying foil, and with increasing coating thickness it becomes even smaller. The RMS slope of cold rolled copper foil (and other metal foils) meet this requirement. Available copper foils for use on printed circuit boards can be used to realize RMS slope values below 0.42 and 0.1. Even smoother copper foils are available GHz range printed circuit boards.

Experimentally, the RMS slope with gold deposited directly on the transducer surface was found to be 2.1, much higher than the value needed to prevent or at least significantly reduce multiple reflections. In contrast, the gold coating of a copper foil resulted in an RMS slope of at most 0.1, sufficiently low to prevent multiple or at least significantly reduce multiple reflections.

In an experiment the photoacoustic amplitude as a function of position on an acoustic-to-electronic transducer and around it was measured by suspending a piece of wall structure in bowl 1, with an acoustic-to-electronic transducer in an opening through that piece of wall structure. The photoacoustic amplitude as a function of position was reconstructed using measurements with acoustic-to-electronic transducers 14 in the basic structure of the bowl. The resulting images show a ring of photoacoustic amplitude around the acoustic-to-electronic transducer in the opening through that piece of wall structure. The root mean square (rms) amplitude of this ring was reduced by about a factor of two when a patches of gold coated copper foil that extends over the gap between the acoustic-to-electronic transducer in the opening and the piece of wall structure. The reduction can be attributed to prevention that light reaches into the gap between the acoustic-to-electronic transducer and the wall structure. The remaining lower rms amplitude ring of photoacoustic amplitude appears to be associated with the edge of the patch. This rms amplitude can be reduced by rounding the edge of the patch.

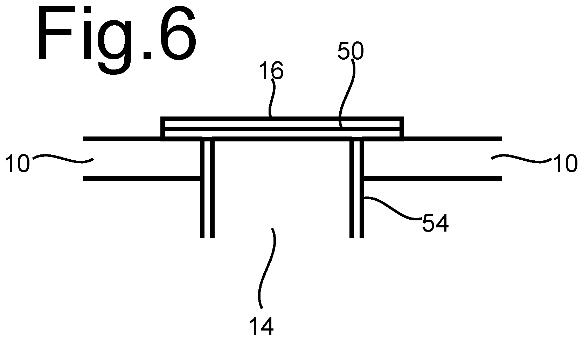

Figure 6 shows an embodiment wherein an electrically conductive layer 50 is provided between the adhesive of patch 16 and the surface of the acoustic-to-electronic transducer 14. Instead, electrically conductive layer 50 may be the copper layer of the patch, with the adhesive layer of the patch 16 being am electrically conductive. Electrically conductive layer 50 or the adhesive layer of the patch 16 is electrically connected to the basic structure 10 of the bowl, which is also electrically conductive. This makes it possible to use the electrically conductive layer 50 and/or the copper foil to shield the transducer 14 from external electromagnetic interference.

Acoustic-electronic transducer 14 may have an electrically conductive housing 54 around a normal axis of the receiving surface. Electrically conductive housing 54. In embodiment housing 54 is electrically connected to electrically conductive layer 50.

**Claims**

1. A photo-acoustic imaging device (1) comprising

   - a basin for receiving tissue to be imaged;
   - a light source configured to supply light to an interior of the basin;
   - a plurality of acoustic-electronic transducers (14), each transducer (14) of said plurality having a sound receiving surface that forms part of a surface of the basin, at a respective different position in the surface of the basin, each with a foil adhesively connected to the sound receiving surface and a reflective coating (42) provided on the basin side of the foil,
   wherein the surface of the basin is formed by a basic structure (10) with openings, the acoustic-electronic transducers (14) extending through the openings of the basic structure (10), the device (1) comprising respective patches (16) of the foils, each over a respective one of said plurality of acoustic-electronic transducers (14), and wherein each of the patches (16) extends from the sound receiving surface of the acoustic-electronic transducer (14) to the surface of the basin around the opening through which the acoustic-electronic transducer extends.

2. The photo-acoustic imaging device of claim 1, wherein the foil is a metal foil and the reflective coating is of a material that is more reflective for the light from the light source than the material of the metal foil.

3. The photo-acoustic imaging device of any one of the preceding claims wherein the reflective coating is a gold coating.

4. The photo-acoustic imaging device of claim 3, wherein the gold coating has a thickness of between 0.1 and 2 micrometers.

5. The photo-acoustic imaging device of any one of the preceding claims wherein the foil is a copper foil.

6. The photo-acoustic imaging device of claim 5, comprising a nickel layer between a gold coating and the copper foil, the nickel layer preferably having a thickness of at least two micrometer.

7. The photo-acoustic imaging device of claim 5 or 6, wherein the copper foil has a thickness of between 1 and 200 micrometer.

8. The photo-acoustic imaging device of any one of the preceding claims, wherein the foil is adhesively connected to the receiving surface by an acrylic glue or a silicon-based glue.

9. The photo-acoustic imaging device of any one of the preceding claims, wherein the acoustic-electronic transducers have an electrically conductive housing around a normal axis of the receiving surface, the foil being a metal foil, the electrically conductive housing being electrically connected to the metal foil.

10. A method of mounting an acoustic-electronic transducer (14) in a photo-acoustic imaging device (1), wherein the photo-acoustic imaging device (1) comprises a basic structure (10) that defines a surface of a basin for receiving tissue to be imaged, the basic structure (10) defining an opening in the surface of the basin, the method comprising

    - mounting the acoustic-electronic transducer (14) at a position where the acoustic-electronic transducer (14) extends through the opening, so that a sound receiving surface of the acoustic-electronic transducer (14) forms part of the surface of the basin; and subsequently
    - adhesively attaching a foil to the sound receiving surface, the foil having a reflective coating (42) on a side of the foil that faces an interior of the basin, wherein the foil is a patch (16) of foil extending from the sound receiving surface of the acoustic-electronic transducer (14) to the surface of the basin around the opening through which the acoustic-electronic transducer (14) extends.

11. The method of claim 10, wherein the metal foil has a pre-applied layer of adhesive on a further side that faces the sound receiving surface.

12. The method of claim 10 or 11, wherein respective foil patches are attached to the sound receiving surfaces of respective acoustic-electronic transducers that extend through all of the openings, each of the respective metal foil patches having a reflective coating on a side of the foil that faces the interior of the basin.


**Patentansprüche**

1. Photoakustisches Bildgebungsgerät (1), umfassend

    - ein Becken zum Empfangen von abzubildendem Gewebe;
    - eine Lichtquelle, die so konfiguriert ist, dass sie Licht in das Innere des Beckens liefert;
    - eine Vielzahl von akustisch-elektronischen Wandlern (14), wobei jeder Wandler (14) der Vielzahl eine Schallempfangsoberfläche aufweist, die einen Teil einer Oberfläche des Beckens bildet, an einer jeweils unterschiedlichen Position in der Oberfläche des Beckens, jeweils mit einer Folie, die mit der Schallempfangsoberfläche mittels Klebstoff verbunden ist, und einer reflektierenden Beschichtung (42), die auf der Beckenseite der Folie vorgesehen ist,

        wobei die Oberfläche des Beckens durch eine Grundstruktur (10) mit Öffnungen gebildet wird, wobei sich die akustisch-elektronischen Wandler (14) durch die Öffnungen der Grundstruktur (10) erstrecken, wobei das Gerät (1) jeweilige Folienpflaster (16) umfasst, die sich jeweils über einem jeweiligen der mehreren akustisch-elektronischen Wandler (14) befinden, und
        wobei sich jedes der Pflaster (16) von der Schallempfangsoberfläche des akustisch-elektronischen Wandlers (14) bis zur Oberfläche des Beckens um die Öffnung herum erstreckt, durch die sich der akustisch-elektronische Wandler erstreckt.

2. Photoakustisches Bildgebungsgerät nach Anspruch 1, wobei die Folie eine Metallfolie ist und die reflektierende Beschichtung aus einem Material besteht, das für das Licht aus der Lichtquelle reflektierender ist als das Material der Metallfolie.

3. Photoakustisches Bildgebungsgerät nach einem der vorstehenden Ansprüche, wobei die reflektierende Beschichtung eine Goldbeschichtung ist.

4. Photoakustisches Bildgebungsgerät nach Anspruch 3, wobei die Goldbeschichtung eine Dicke zwischen 0,1 und 2 Mikrometern aufweist.

5. Photoakustisches Bildgebungsgerät nach einem der vorstehenden Ansprüche, wobei die Folie eine Kupferfolie ist.

6. Photoakustisches Bildgebungsgerät nach Anspruch 5, umfassend eine Nickelschicht zwischen einer Goldbeschichtung und der Kupferfolie, wobei die Nickelschicht vorzugsweise eine Dicke von mindestens zwei Mikrometern aufweist.

7. Photoakustisches Bildgebungsgerät nach Anspruch 5 oder 6, wobei die Kupferfolie eine Dicke zwischen 1 und 200 Mikrometern aufweist.

8. Photoakustisches Bildgebungsgerät nach einem der vorstehenden Ansprüche, wobei die Folie durch einen Acrylkleber oder einen Kleber auf Silikonbasis mit der Empfangsfläche mittels Klebstoff verbunden ist.

9. Photoakustisches Bildgebungsgerät nach einem der vorstehenden Ansprüche, wobei die akustisch-elektronischen Wandler ein elektrisch leitfähiges Gehäuse um eine Normalenachse der Empfangsfläche herum aufweisen, wobei die Folie eine Metallfolie ist und das elektrisch leitfähige Gehäuse elektrisch mit der Metallfolie verbunden ist.

10. Verfahren zum Anbringen eines akustisch-elektronischen Wandlers (14) in einem photoakustischen Bildgebungsgerät (1), wobei das photoakustische Bildgebungsgerät (1) eine Grundstruktur (10) umfasst, die eine Oberfläche eines Beckens zum Aufnehmen von abzubildendem Gewebe definiert, wobei die Grundstruktur (10) eine Öffnung in der Oberfläche des Beckens definiert, wobei das Verfahren umfasst

   - Anbringen des akustisch-elektronischen Wandlers (14) an einer Position, an der sich der akustisch-elektronische Wandler (14) durch die Öffnung erstreckt, sodass eine Schallempfangsoberfläche des akustisch-elektronischen Wandlers (14) einen Teil der Oberfläche des Beckens bildet; und anschließend
   - Anbringen einer Folie auf der Schallempfangsoberfläche mittels Klebstoff, wobei die Folie eine reflektierende Beschichtung (42) auf einer Seite der Folie aufweist, die zum Inneren des Beckens hin ausgerichtet ist, wobei die Folie ein Folienpflaster (16) ist, das sich von der Schallempfangsoberfläche des akustisch-elektronischen Wandlers (14) bis zur Oberfläche des Beckens um die Öffnung herum erstreckt, durch die sich der akustisch-elektronische Wandler (14) erstreckt.

11. Verfahren nach Anspruch 10, wobei die Metallfolie auf einer weiteren Seite, die der Schallempfangsoberfläche zugewandt ist, eine vorab aufgebrachte Klebeschicht aufweist.

12. Verfahren nach Anspruch 10 oder 11, wobei jeweilige Folienpflaster an den Schallempfangsoberflächen jeweiliger akustisch-elektronischer Wandler angebracht sind, die sich durch alle Öffnungen erstrecken, wobei jedes der jeweiligen Metallfolienpflaster eine reflektierende Beschichtung auf einer Seite der Folie aufweist, die zum Inneren des Beckens zeigt.

**Revendications**

1. Dispositif d'imagerie photo-acoustique (1) comprenant

   - une cuve destinée à recevoir un tissu à mettre en image ;
   - une source lumineuse configurée pour fournir une lumière à un intérieur de la cuve ;
   - une pluralité de transducteurs acoustico-électroniques (14), chaque transducteur (14) parmi ladite pluralité présentant une surface de réception de son qui constitue une partie d'une surface de la cuve, en une position différente respective dans la surface de la cuve, chacune avec une feuille reliée de façon adhésive à la surface de réception de son et un revêtement réfléchissant (42) prévu sur le côté de cuve de la feuille,

   dans lequel la surface de la cuve est constituée par une structure de base (10) avec des ouvertures, les transducteurs acoustico-électroniques (14) s'étendant à travers les ouvertures de la structure de base (10), le dispositif (1) comprenant des plaques (16) des feuilles, chacune par-dessus un transducteur acoustico-électronique respectif parmi ladite pluralité de transducteurs acoustico-électroniques (14), et dans lequel chacune des plaques (16) s'étend depuis la surface de réception de son du transducteur acoustico-électronique (14) vers la surface de la cuve autour de l'ouverture à travers laquelle le transducteur acoustico-électronique s'étend.

2. Dispositif d'imagerie photo-acoustique selon la revendication 1, dans lequel la feuille est une feuille métallique et le revêtement réfléchissant est fabriqué dans un matériau qui est plus réfléchissant pour la lumière depuis la source lumineuse que le matériau de la feuille métallique.

3. Dispositif d'imagerie photo-acoustique selon l'une quelconque des revendications précédentes, dans lequel le revêtement réfléchissant est un revêtement d'or.

4. Dispositif d'imagerie photo-acoustique selon la revendication 3, dans lequel le revêtement d'or présente une épaisseur de 0,1 à 2 micromètres.

5. Dispositif d'imagerie photo-acoustique selon l'une quelconque des revendications précédentes, dans lequel la feuille est une feuille de cuivre.

6. Dispositif d'imagerie photo-acoustique selon la revendication 5, comprenant une couche de nickel entre un revêtement d'or et la feuille de cuivre, la couche de nickel présentant de préférence une épaisseur d'au moins deux micromètres.

7. Dispositif d'imagerie photo-acoustique selon la revendication 5 ou 6, dans lequel la feuille de cuivre présente une épaisseur de 1 à 200 micromètres.

8. Dispositif d'imagerie photo-acoustique selon l'une quelconque des revendications précédentes, dans lequel la feuille est reliée de façon adhésive à la surface de réception par une colle acrylique ou une colle à base de silicium.

9. Dispositif d'imagerie photo-acoustique selon l'une quelconque des revendications précédentes, dans lequel les transducteurs acoustico-électroniques présentent un logement électriquement conducteur autour d'un axe normal de la surface de réception, la feuille étant une feuille métallique, le logement électriquement conducteur étant électriquement relié à la feuille métallique.

10. Procédé de montage d'un transducteur acoustico-électronique (14) dans un dispositif d'imagerie photo-acoustique (1), dans lequel le dispositif d'imagerie photo-acoustique (1) comprend une structure de base (10) qui définit une surface d'une cuve destinée à recevoir un tissu à mettre en image, la structure de base (10) définissant une ouverture dans la surface de la cuve, le procédé comprenant :

    - le montage du transducteur acoustico-électronique (14) en une position où le transducteur acoustico-électronique (14) s'étend à travers l'ouverture, de sorte qu'une surface de réception de son du transducteur acoustico-électronique (14) constitue une partie de la surface de la cuve ; et ensuite
    - la fixation adhésive d'une feuille contre la surface de réception de son, la feuille présentant un revêtement réfléchissant (42) sur un côté de la feuille qui fait face à un intérieur de la cuve, dans lequel la feuille est une plaque (16) de feuille s'étendant depuis la surface de réception de son du transducteur acoustico-électronique (14) vers la surface de la cuve autour de l'ouverture à travers laquelle le transducteur acoustico-électronique (14) s'étend.

11. Procédé selon la revendication 10, dans lequel la feuille métallique présente une couche pré-appliquée d'adhésif sur un autre côté qui fait face à la surface de réception de son.

12. Procédé selon la revendication 10 ou 11, dans lequel des plaques de feuille respectives sont fixées contre les surfaces de réception de son de transducteurs acoustico-électroniques respectifs qui s'étendent à travers toutes les ouvertures, chacune parmi les plaques de feuille métallique présentant un revêtement réfléchissant sur un côté de la feuille qui fait face à l'intérieur de la cuve.

# Fig.1

10

1

12

14

# Fig.3

14

16

# Fig.2

16

16

16

10

14

14

14

# Fig.4

# Fig.5

# Fig.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019275562 A
- US 20170030866 A1
- US 2018292358 A1
- US 2017059530 A1

**Non-patent literature cited in the description**

- Properties of Metals. **PAQUIN, R.E**. Handbook of Optics. McGraw-Hill Professional, 1995, vol. II, 35.1-35.74
- Mirror and Reflector Materials. **LYNCH, D.W.** CRC Handbook of Laser Science and Technology. CRC Press, 2003
- **CALLENS et al.** Matching ultrasonic transducer using two matching layers where one of them is glue. *NDT&E International*, 2004, vol. 37, 591-597
- **BERGSTROM**. The absorption of light by rough metal surfaces_ a three-dimensional ray-tracing analysis. *Journal of Applied Physics*, 2008, vol. 103, 103515